# EUROPEAN PATENT SPECIFICATION

(11) **EP 2 413 988 B1**
(45) Date of publication and mention of the grant of the patent: **04.03.2015**
(21) Application number: 10722452.9
(22) Date of filing: 24.03.2010
(51) Int. Cl.: A61L 31/10, A61L 31/14

(54) **INTRALUMINAL DEVICE WITH CONTROLLED BIODEGRADATION**
INTRALUMINALE VORRICHTUNG MIT KONTROLLIERTEM BIOLOGISCHEN ABBAU
DISPOSITIF INTRALUMINAL AVEC BIODÉGRADATION CONTRÔLÉE

(30) Priority: 30.03.2009 US 414144
(43) Date of publication of application: 08.02.2012
(73) Proprietor: Cook Medical Technologies LLC, Bloomington, IN 47404 (US)
(72) Inventor: GIBBONS, William, S., Jr., Winston-Salem NC 27104 (US); LU, Wenfeng, Pfafftown, North Carolina 27040 (US)
(74) Representative: Garratt, Peter Douglas
(86) International application number: PCT/US2010/028438
(87) International publication number: WO 2010/117629

(56) References cited:
- WO-A1-2004/110515
- DE-A1- 19 945 049

## Description

### BACKGROUND

The present disclosure relates to a biodegradable intraluminal device, such as an intraluminal stent, that undergoes biodegradation within a body lumen. Biodegradable devices such as intraluminal stents are currently implanted in various body lumens of a patient, including the coronary vasculature, the tracheal tract, and the gastrointestinal tract. In certain situations, it may be desirable for the stent to be biodegradable or bioabsorbable so as to reduce the adverse risks that would otherwise be associated with the stent's continued presence once its usefulness at the treatment site has ceased or has at least become substantially diminished. To such end, some stents have heretofore been wholly constructed of materials that are biodegradable or bioabsorbable. It is desirable to select a material that while biodegradable is nonetheless biocompatible and has sufficient strength to support the loads a particular stent will be subjected to when implanted.

When the stent is implanted within the target body region, the inner and outer surfaces of the biodegradable stent contact bodily fluids which cause the onset of biodegradation of the implanted stent. As the stent biodegrades in vivo, the stent loses mass. Oftentimes, the rate of mass loss can be significant and becomes difficult to regulate and control. The significant loss in mass can lead to a premature loss in mechanical strength of the stent, thereby rendering the stent incapable of maintaining the patency of the target body lumen for its intended time frame.

### SUMMARY

In a first aspect, a hybrid degradable stent is provided. The hybrid stent comprises a generally tubular main body comprising an inner diameter and an outer diameter. The tubular body is formed from a biodegradable material. The hybrid stent further comprises a photodegradable layer disposed over at least a portion of the inner diameter and/or the outer diameter of the biodegradable tubular body. The layer is formed from a photodegradable material that is chemically inert to bodily fluids contained at an implanted site. The photodegradable layer is selectively adapted to be activated from a chemically inert state to a photodegradable state. The photodegradable state initiates degradation of the photodegradable layer so as to expose at least a portion of the biodegradable material to begin biodegradation of the biodegradable material.

In a second aspect, a degradable stent kit is provided. The kit comprises a generally biodegradable tubular body comprising a proximal end and a distal end, and a lumen extending from the proximal end to the distal end. The body further comprises a photodegradable material disposed over at least a portion of the biodegradable tubular body. The photodegradable material is chemically inert when deployed into a body lumen of a patient. The kit also includes a light-irradiating system configured to activate the photodegradable material from the chemically inert state to a photodegradable state. The light-irradiating system comprises a light source and a fiber section. The fiber section comprises a proximal section in communication with the light source and a distal section in communication with the tubular body. The fiber section is adapted to propagate UV light from the proximal section to the distal section and thereafter irradiate UV light from the distal section to the photodegradable material.

### BRIEF DESCRIPTION OF THE DRAWINGS

Figure 1a shows a cross-sectional view of a hybrid biodegradable stent having an outer photodegradative layer and an inner photodegradative layer over the biodegradable stent;
Figure 1b shows a perspective view of the hybrid biodegradable stent of Figure 1a;
Figure 2a shows a partial cross-sectional view of an optical fiber inserted within a luminal space of the hybrid biodegradable stent of Figure 1a, the optical fiber propagating ultraviolet light towards the inner photodegradative layer so as to activate and begin the degradation process of the inner photodegradative layer;
Figure 2b shows a partial cross-sectional view of the optical fiber inserted within the luminal space of the hybrid biodegradable stent of Figure 1 in which a portion of the inner photodegradable layer has been activated, degraded, and removed with the optical fiber continuing to propagate ultraviolet light towards the inner photodegradative layer so as to activate, degrade, and remove a further portion of the inner photodegradative layer;
Figure 2c shows a partial cross-sectional view of the optical fiber inserted within the luminal space of the hybrid biodegradable stent of Figure 1a in which all of the inner photodegradative layer has been removed within the inner surfaces of the biodegradable stent;
Figure 3a shows a partial cross-sectional view of the optical fiber now positioned outside of luminal space of biodegradable stent along a proximal edge of stent, the optical fiber propagating ultraviolet light towards the outer photodegradative layer so as to initiative activation and begin degradation of the outer photodegradative layer along the outer surface of the biodegradable stent;
Figure 3b shows a partial cross-sectional view of the optical fiber positioned outside of luminal space of biodegradable stent and slightly advanced distally along an outer surface of stent in which a portion of the outer photodegradable layer has been activated, degraded, and removed with the optical fiber continuing to propagate ultraviolet light towards the outer photodegradative layer so as to further activate, degrade, and remove the outer photodegradative layer along the outer surfaces of the biodegradable stent;
Figure 3c shows a partial cross-sectional view of the optical fiber outside of luminal space of biodegradable stent and advanced further distally along the outer surface of the stent so as to photodegrade and remove all of the outer photodegradative layer from the outer surfaces of the biodegradable stent;
Figure 4a shows a lens affixed to the distal end of the optical fiber, the lens redirecting the UV waves in a first direction normal to the longitudinal axis of the optical fiber; and
Figure 4b shows the optical fiber rotated so as to reconfigure the lens such that the UV waves are redirected in a second direction normal to the longitudinal axis of the optical fiber.

### DETAILED DESCRIPTION

The relationship and functioning of the various elements of this invention are better understood by the following detailed description. However, the embodiments of this invention as described below are by way of example only. It should also be understood that the drawings are not to scale and in certain instances details, which are not necessary for an understanding of the present invention, have been omitted such as conventional details of fabrication and assembly. Unless otherwise specified, all percentages expressed herein are weight percentages based on the whole mixture.

The term "biodegradable" material refers to a material that dissipates upon implantation within a body, independent of the mechanisms by which dissipation can occur, such as dissolution, degradation, absorption and excretion. The actual choice of which type of materials to use may readily be made by one of ordinary skill in the art. Such materials are often referred to by different terms in the art, such as "bioresorbable," "bioabsorbable," or "biodegradable," depending upon the mechanism by which the material dissipates. The prefix "bio" indicates that the erosion occurs under physiological conditions, as opposed to other erosion processes, caused for example, by high temperature, strong acids or bases, or ultraviolet ("UV") light.

The terms "proximal" and "distal" as used herein are intended to have a reference point relative to the user. Specifically, throughout the specification, the terms "distal" and "distally" shall denote a position, direction, or orientation that is generally away from the user, and the terms "proximal" and "proximally" shall denote a position, direction, or orientation that is generally towards the user.

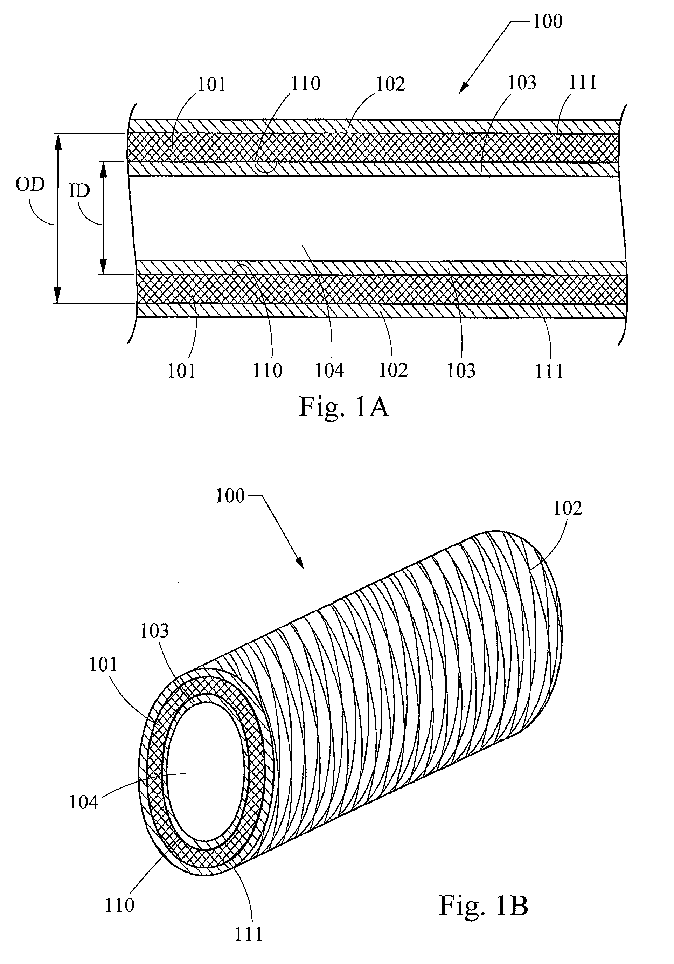

Figures 1A and 1B show a cross-sectional view of an exemplary hybrid degradable stent 100. The hybrid degradable stent 100 comprises a biodegradable stent 101, which is the tubular main body, disposed between an outer photodegradable layer 102 and an inner photodegradable layer 103. Figure 1A shows that the tubular main body is characterized as having an inner diameter "ID" and an outer diameter "OD". The inner photodegradable layer 103 extends along the "ID" at an inner surface 110 of stent 101. The outer photodegradable layer 102 extends along the "OD" at an outer surface 111 of stent 101. The inner photodegradable surface 103 preferably extends along the entire longitudinal length of the stent 101 over inner surface 110. Similarly, the outer photodegradable layer 102 preferably extends along the entire longitudinal length of the stent 101 over outer surface 111. As used herein, the term "layer" refers to any means by which material may be disposed along an inner surface 110 and/or an outer surface 111 of the biodegradable stent 101. The inner and the outer photodegradable layers 103 and 102 are chemically inert when in contact with bodily fluids at the implanted site. In other words, the photodegradable layers are not susceptible to any type of biodegradation breakdown that typically occurs when biodegradable materials are exposed to bodily fluids at the implanted site. Preferably, the inner and the outer photodegradable layers 103 and 102 are nonporous and extend along an entire longitudinal length of the stent 101. Accordingly, the inner and the outer photodegradable layers 103 and 102 serve as a protective outer covering or sheath over the biodegradable stent 101 when implanted. When patency of the target body lumen by stent 100 is no longer required, degradation of the protective photodegradable layers 102 and 103 can begin. UV light or another type of activation agent contacts the photodegradable layers 102 and 103, which causes the photodegradable layers 102 and 103 to become activated and thereafter degrade. As the inner photodegradable layer 103 and the outer photodegradable layer 102 degrade, they detach from biodegradable stent 101 thereby causing at least a portion of the inner surface 110 and outer surface 111 of the biodegradable stent 101 to be exposed. Exposure of outer surface 111 to the implanted environment begins biodegradation of the stent 100 along the outer surface 111. Similarly, exposure of inner surface 110 to the implanted environment begins biodegradation of the stent 100 along the inner surface 120. Accordingly, the biodegradation of stent 101 can be delayed for a predetermined length of time by maintaining protective photodegradable layers 102 and 103 on biodegradable stent 101, thereby avoiding premature biodegradation of biodegradable stent 101.

A variety of photodegradable materials may be used. For example, the photodegradable layer may be a blend of polymers, which includes a base or synthetic polymer and small amount of UV photodegradable ketocarbonyl containing polymer. The amount of keto carbonyl groups in the composition may range from about 0.01 wt% to about 5 wt%, based upon the total weight of the base polymer. The keto carbonyl group is a ketone functional group characterized by a carbonyl group (O=C) linked to two other carbon atoms. The keto carbonyl group can be generally designated as R₁(CO)R₂.

The base or synthetic polymer may comprise a vinylidene monomer which is compatible with the keto carbonyl groups. "Compatible" as used herein refers to polymers which can be blended together in the desired proportions to give a polymer blend of reasonable strength and toughness. The vinylidene monomer may comprise ethylene, styrene, methyl acrylate, methyl methacrylate, vinyl acetate, methacrylonitrile, acrylonitrile, vinyl chloride, acrylic acid, methacrylic acid, chlorostyrene, alpha-methylstyrene, vinyl toluene or butadiene. In one example, a blend of polyethylene and about 9.5 wt% methylenemethyl isopropenyl ketone copolymer may be utilized. The polyethylene may be low density or high density. In another example, a copolymer of 95 wt% styrene and 5 wt% methylisopenylketone may be utilized.

The polymeric composition may also include a condensation copolymer and at least one ketone copolymer in which the amount of the ketone copolymer ranges from about 0.01 wt% to about 5 wt%. The condensation copolymer may comprise polyamides, polyesters, polyurethanes, polyethers, polyeopxides, polyamide esters, polyimides, poly(amide-imides), polyureas, and polyamino-acids.

It is preferred to choose an addition copolymer of a similar vinylidene monomer and an unsaturated ketone, in minor proportion. It is especially preferred to use a minor proportion of a UV photodegradable copolymer based upon one of the monomers of a synthetic polymer. For example, among the especially preferred embodiments are such compositions as blends of polystyrene (major proportion) and keto-carbonyl containing copolymers of styrene (minor proportion), blends of polymethylmethacrylate (major proportion) and keto-carbonyl containing copolymers of methyl-methacrylate (minor proportion), blends of polymethylacrylate (major proportion) and keto-carbonyl containing copolymers of methylacrylate (minor proportion), and blends of polyethylene (major proportion) and keto carbonyl containing ethylene - unsaturated ketone copolymers (minor proportion), being macro-molecular. The amount of keto carbonyl groups in the composition may range from about 0.01 wt% to about 5 wt%.

The keto copolymers used in minor proportion in the preferred compositions of the present invention are themselves photodegradable on exposure to UV radiation. They may contain from about 0.01 - 10 wt%, preferably from about 0.01 - 5 wt%, and most preferably from about 0.02 - 2 wt% of a ketone carbonyl group. They are compatible with the base polymer (i.e., the synthetic polymer) with which they are to be blended. In the case of addition keto copolymers, the keto groups are located in a side chain at a position immediately adjacent to the backbone polymeric chain. In the case of condensation keto copolymers, the keto groups may be located either in a side chain as mentioned above, or in the polymer backbone. The keto copolymer is blended with the synthetic polymer so as to give a polymeric composition preferably containing not more than 3 wt% keto groups in these preferred compositions.

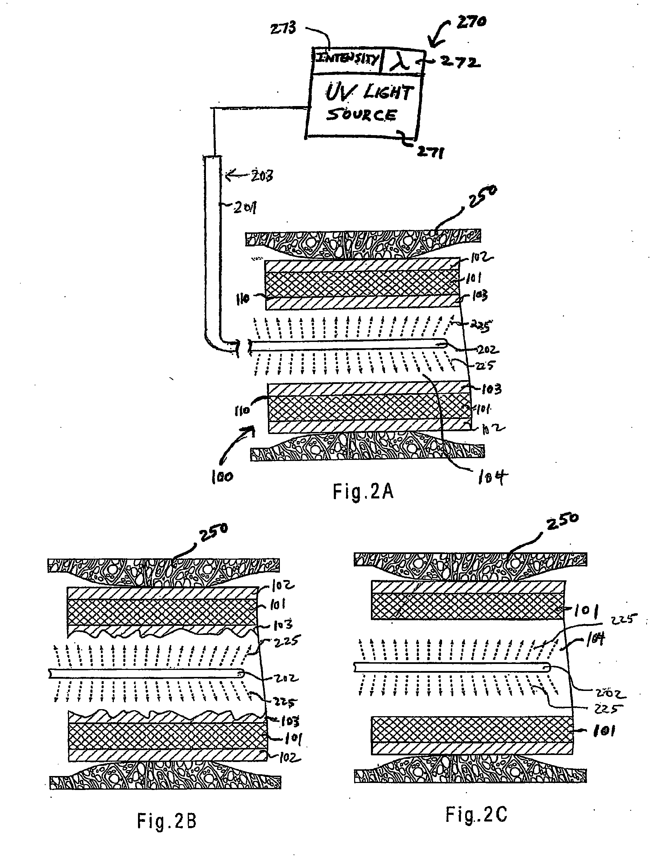

A preferred means for activating degradation of outer photodegradable layer 102 and inner photodegradable layer 103 is by a light irradiating system 270 (Figure 2). Referring to Figure 2, the light irradiating system 270 comprises a UV light source 271 and an optical fiber section 203. The intensity 273 or power of the UV light 225 emitted into proximal region 201 of the optical fiber 203 may be regulated by UV irradiation system 270. UV light is delivered from the UV light source 271 through optical fiber 203. The optical fiber 203 is constructed to enable transmission of the high-energy UV light with minimal attenuation of its intensity 273. Specifically, the proximal region 201 of optical fiber 203 is preferably encased in a jacketing material known in the art (e.g., fused silica) to allow propagation of the UV light waves therethough without significant attenuation or leakage. The distal region 202 of the optical fiber 203 constitutes the light transmission section along which the UV light waves 225 are emitted towards the inner photodegradable layer 103. The distal region 202 extends towards the inner photodegradable layer 103 (Figures 2a-2c). Figure 2a shows that the proximal region 201 of optical fiber 203 is coupled to the UV light source 271 of a suitable wavelength to interact with the inner photodegradable material of layer 103. The radiation source 271 can include an intensity control 273 to regulate the amount of UV light to be delivered to the optical fiber 203. The UV source 271 can also include a wavelength tuning or selection control 272 for selecting radiation of a suitable wavelength to interact with the photodegradable material of the outer layer 102 and the inner layer 103.

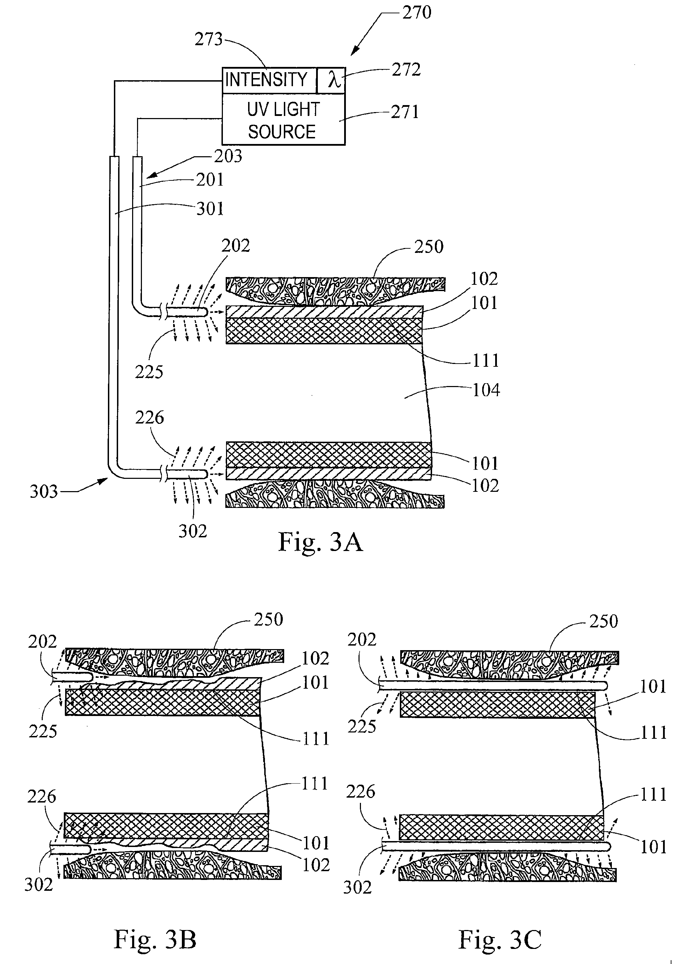

After the hybrid stent 100 has maintained the patency of a body lumen 250 for the desired time frame, biodegradation of the stent 101 may ensue, which involves degrading the protective inner photodegradative layer 103 and the protective outer photodegradative layer 102. Figures 2A-2C and 3A-3C show the sequence of steps involved in activating, degrading and removing the photodegradable layers 103 and 102 from the inner surface 110 and subsequently the outer surface 111 of stent 101 at a target site within body lumen 250.

Accordingly, Figures 2a-2c show the optical fiber 203 positioned within the lumen 104 of the stent 100 body. Navigating the optical fiber 203 to the stent 100 may be facilitated by insertion through a sheath or overtube (not shown). Upon the optical fiber 203 reaching the target site of body lumen 250, at least the distal region 202 of the optical fiber 203 may be withdrawn from the sheath or overtube.

The distal region 202 of the optical fiber 203 is preferably designed to diffuse UV light 225 outwardly towards the inner surface 110 of stent 101. Figure 2a shows a partial cross-sectional view of the optical fiber 203 inserted within a luminal space 104 of the hybrid biodegradable stent 100 of Figure 1a. The proximal region 201 of the optical fiber 203 extends outside of a patient and is connected to the UV source 271 of UV light irradiating system 270. A suitable wavelength 272 and intensity 273 of UV light 225 is selected from the UV light source 271. The UV light 225 propagates through proximal portion 201 of the optical fiber 203 into distal portion 202 of optical fiber 203. The UV light 225 diffuses towards the inner photodegradative layer 103 so as to activate and begin the degradation process of the inner photodegradative layer 103.

The distal end of the optical fiber 203 can include a divergent lens shape which can disperse the UV light 225 radially outward from the axis of the optical fiber 203 in all directions towards the inner photodegradable layer 103. The UV light 225 diffuses in all directions towards inner photodegradable layer 103 such that the UV irradiance is approximately uniform along the longitudinal length of the stent 101, as shown in Figures 2A-2C. Alternatively, the distal tip of the optical fiber 203 may be tapered to diffuse the UV light waves 225 outwardly towards the inner photodegradable surface 103. The duration for UV irradiation is sufficient for activation of the inner photodegradable material 103 to occur. The duration of the UV light irradiation will depend at least in part on the particular inner photodegradable material 103 used, the concentration of the ketocarbonyl groups contained within inner photodegradable layer 103, and the power or intensity 273 emitted from UV light irradiation system 270. In one example, the time of UV irradiation is about a minute.

There is a predetermined amount of energy associated with the UV light 225 at the predetermined wavelength and intensity. As the UV light 225 contacts the inner photodegradable layer 103, this energy is sufficient to activate the inner photodegradable layer 103 such that the onset of cleavage of the chemical bonds of the polymer chains of the UV photodegradable layer 103 occurs. The emitted UV light 225 is absorbed by layer 103. The absorption of the UV light 225 results in added thermal energy which is sufficient to cleave the chemical bonds of the polymer chains of inner photodegradable layer 103. Preferably, the UV light 225 raises the temperature of the inner photodegradable layer 103 to above its glass transition temperature. The material of layer 103 begins to disintegrate and degrades to a point where the inner photodegradable material 103 becomes detached from the inner surface 110 of the stent 101. Figure 2b shows that a portion of the inner photodegradable layer 103 has been activated, degraded, and removed as UV light 225 contacts the inner photodegradable layer 103.

UV light 225 continues to propagate through distal region 202 of the optical fiber 230 so as to further activate and degrade a portion of the inner photodegradable layer 103, thereby removing an additional portion of layer 103 from inner surface 110. Figure 2c shows that all of the inner photodegradable layer 103 has been removed so as to expose inner surface 110 of stent 101 to its implanted environment at the body lumen 250. Exposure of the inner surface 110 of stent 101 may enable biodegradation of stent 101 to now occur.

After the inner photodegradable layer 103 has been removed from inner surface 110 of stent 101, the optical fiber 203 may be re-positioned along the outer surface 111 of the stent 101 as shown in Figures 3A-3C to remove outer photodegradable material 102 along outer surface 111 of stent 101. The proximal region 201 of the optical fiber 203 extends outside of a patient and is connected to the UV source 271 of UV light irradiating system 270. A suitable wavelength 272 and intensity 273 of UV light 225 is selected from the UV light source 271. The UV light 225 propagates through proximal portion 201 of the optical fiber 203 into distal portion 202 of optical fiber 203. The UV light 225 is emitted and diffuses towards the outer photodegradative layer 102 so as to activate and begin the degradation process of the outer photodegradative layer 102.

Figure 3a also shows that a second optical fiber 303 may be positioned along the outer surface 111 of the stent 101 in which the second optical fiber 303 is disposed about 180° relative to the optical fiber 203. A suitable wavelength 272 and intensity 273 of UV light 226 is selected from the UV light source 271. The UV light 226 propagates through proximal portion 301 of the optical fiber 303 into distal portion 302 of optical fiber 303. The UV light 226 is emitted and diffuses towards the outer photodegradative layer 102 so as to activate and begin the degradation process of the outer photodegradative layer 102 along the bottom portion of stent 101. Having a second optical fiber 303 simultaneously remove material from outer photodegradable layer 102 eliminates the need to reposition the optical fiber 203 along the outer surface 111 of stent 101, and therefore may reduce the time needed for irradiating UV light from UV irradiating system 270. Figure 3a shows that UV light 225 emitted and diffused from optical fiber 203 and UV light 226 emitted and diffused from optical fiber 303 contact outer photodegradable layer 102 so as to initiative activation and begin degradation of the outer photodegradative layer 102.

Exposure of the outer photodegradable material 102 to UV light waves 225 and 226 may be emitted in all directions, as shown in Figures3a-3c. Figure 3b shows that a portion of the outer layer 102 has been degraded and removed along the outer surface 111 from the stent 101. As a result, the distal portions 202 and 302 of the optical fibers 203 and 303 may be slightly advanced distally along the outer surface 111 of stent 101(Figures 3B and 3C) so as to be in close proximity to that portion of the outer photodegradable layer 102 still affixed to the outer surface 111 of stent 101. UV light 225 continues to propagate through optical fiber 203 and UV light 226 continues to propagate through optical fiber 303 to further activate, degrade, and remove additional amounts of the outer photodegradative layer 102. Propagation of UV light 225 and 226 to outer layer 102 preferably continues until all of the outer photodegradable layer 102 has been removed so as to expose outer surface 111 of stent 101 to its implanted environment at the body lumen 250. Exposure of the outer surface 111 of stent 101 may enable biodegradation to occur along the outer surface 111 of stent 101.

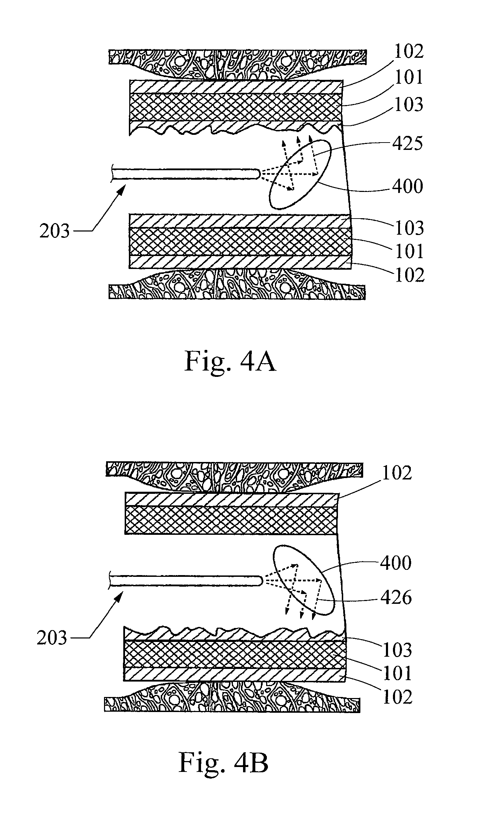

In an alternative embodiment, the distal end of the optical fiber 203 may be fitted or fabricated with a means for selectively directing the UV light waves onto the inner surface 110 of the inner photodegradable layer 103. As an example, Figure 4a shows a lens 400 which may be affixed to the distal tip of the fiber 203 for redirecting the UV light waves in a direction approximately normal to the longitudinal axis of the optical fiber 203. Such a configuration may provide a larger fraction of UV waves 425 propagating towards the top portion of the inner photodegradable layer 103. In other words, the UV waves 425 are redirected in the direction of the surface of the lens 400. Figure 4b shows that the optical fiber 203 may be rotated so as to angle the lens 400 downwards. Such a configuration may provide a larger fraction of UV waves 426 propagating towards the bottom portion of the inner photodegradable layer 103. Such selective redirecting of the UV waves may also be utilized when activating, degrading, and removing portions of the outer photodegradable layer 102.

The distal end of the optical fibers 203 and/or 303 can have various other attachments that will effect this purpose or alternatively the optical fibers 203 and/or 303 themselves can be fabricated so that it side-fires at its end. For example, in addition to the lens surface 400 described above in Figures 4a and 4b, the distal tip of the optical fiber 203 and/or 303 can be made side-firing by beveling the end at an angle of approximately 45° and then applying a mirrored surface to the beveled end. Another example of a side-firing end of the optical fiber 203 and/or 303 can be made by creating a concave cone-shaped recess within the end of the optical fiber 203 and/or 303, and then applying a mirrored surface to the concave surface. In addition to these examples, many other optical attachments and means of modifying the end of the optical fiber 203 and/or 303 may be utilized as known in the art. Several other optical means that are well known to achieve this redirection which involve reflection of a mirrored concave cone-shaped recess, mirrored bevels, and defracting and refracting means may also be used.

Variations for removal of the inner photodegradable layer 103 and the outer photodegradable layer 102 are contemplated. For example, the sequence of removal of the inner and outer photodegradable layers 103 and 102 are interchangeable such that the outer photodegradable layer 102 may be removed before the inner photodegradable layer 103. As another example, only a portion of the inner and outer photodegradable layers 103 and 102 may be activated, degraded, and removed.

Although the above described method shows both an inner and outer photodegradable layers 103 and 102, the stent 100 may comprise a single protective layer. In particular, the stent 100 may comprise either an inner photogdegradable layer 103 or an photodegradable layer 102.

Although optical fibers have been shown, other energy conducting means as known in the art may be used to propagate and transmit the UV waves from UV light source 271 to the photodegradable inner layer 103 and the photodegradable outer layer 102.

Various biodegradable polymeric materials may be used to form stent 101. The biodegradable polymer may comprise a polylactic acid (PLA), which may be a mixture of enantiomers typically referred to as poly-D, L-lactic acid. PLA is one of the poly-α-hydroxy acids, which may be polymerized from a lactic acid dimer. This polymer has two enantiomeric forms, poly(L-lactic acid) (PLLA) and poly(D-lactic acid) (PDLA), which differ from each other in their rate of biodegradation. PLLA is semicrystalline, whereas PDLA is amorphous, which may be desirable for applications such as drug delivery where it is important to have a homogeneous dispersion of an active species. PLA has excellent biocompatibility and slow degradation, is generally more hydrophobic than polyglycolic acids (PGA). The polymer used may also desirably comprise polyglycolic acids (PGA). Polyglycolic acid is a simple aliphatic polyester that has a semi-crystalline structure, fully degrades in 3 months, and undergoes complete strength loss by 1 month. Compared with PLA, PGA is a stronger acid and is more hydrophilic, and, thus, more susceptible to hydrolysis.

Other desirable biodegradable polymers for use include, but are not limited to, polylactic glycolic acids (PLGA) and other copolymers of PLA and PGA. The properties of the copolymers can be controlled by varying the ratio of PLA to PGA. For example, copolymers with high PLA to PGA ratios generally degrade slower than those with high PGA to PLA ratios.

Still other desirable polymers for use include poly(ethylene glycol) (PEG), polyanhydrides, polyorthoesters, fullerene, polytetrafluoroethylene, poly(styrene-b-isobutylene-b-styrene), polyethylene-co-vinylacetate, poly-N-butylmethacrylate, amino acid-based polymers (such as poly(ester) amide), SiC, TiNO, Parylene C, heparin, porphorylcholine.

A number of biodegradable homopolymers, copolymers, or blends of biodegradable polymers are known in the medical arts. These include, but are not limited to: polyethylene oxide (PEO), polydioxanone (PDS), polypropylene fumarate, poly(ethyl glutamate-co-glutamic acid), poly(tert-butyloxy-carbonylmethyl glutamate), polycaprolactones (PCL), polyhydroxybutyrates (PHBT), polyvalerolactones, polyhydroxyvalerates, poly(D,L-lactide-co-caprolactone) (PLA/PCL), polycaprolactone-glycolides (PGA/PCL), poly(phosphate ester), and poly(hydroxy butyrate), polydepsipeptides, maleic anhydride copolymers, polyphosphazenes, polyiminocarbonates, polyhydroxymethacrylates, polytrimethylcarbonates, cyanoacrylate, polycyanoacrylates, hydroxypropylmethylcellulose, polysaccharides (such as hyaluronic acid, chitosan and regenerate cellulose), fibrin, casein, and proteins (such as gelatin and collagen), poly-e-decalactones, polylactonic acid, polyhydroxybutanoic acid, poly(1,4-dioxane-2,3-diones), poly(1,3-dioxane-2-ones), poly-p-dioxanones, poly-b-maleic acid, polycaprolactonebutylacrylates, multiblock polymers, polyether ester multiblock polymers, poly(DTE-co-DT-carbonate), poly(N-vinyl)-pyrrolidone, polyvinylalcohols, polyesteramides, glycolated polyesters, polyphosphoesters, poly[p-carboxyphenoxy)propane], polyhydroxypentanoic acid, polyethyleneoxide-propyleneoxide, polyurethanes, polyether esters such as polyethyleneoxide, polyalkeneoxalates, lipides, carrageenanes, polyamino acids, synthetic polyamino acids, zein, polyhydroxyalkanoates, pectic acid, actinic acid, carboxymethylsulphate, albumin, hyaluronic acid, heparan sulphate, heparin, chondroitinesulphate, dextran, b-cyclodextrines, gummi arabicum, guar, collagen-N-hydroxysuccinimide, lipides, phospholipides, resilin, and modifications, copolymers, and/or mixtures of any of the carriers identified herein.

Other suitable biodegradable polymers that may be used include, but are not limited to: aliphatic polyesters (including homopolymers and copolymers of lactide), poly(lactide-co-glycolide), poly(hydroxybutyrate-co-valerate), poly(hydroxybutyrate-co-hydroxyvalerate) (PHBV), polyoxaster and polyoxaesters containing amido groups, polyamidoester, poly(glycolic acid-co-trimethylene carbonate), poly(trimethylene carbonate), and biomolecules and blends thereof such as fibrinogen, starch, elastin, fatty acids (and esters thereof), glucoso-glycans, and modifications, copolymers, and/or mixtures or combinations of any of the carriers identified herein.

The hybrid stent 100 may be created by coating the biodegradable stent 101 in any way known in the art. As an example, the biodegradable stent 101 may be extruded as known in the art. The inner photodegradable layer 103 may be coated onto the inner surface 110, and the outer photodegradable layer 102 may be coated onto the outer surface 111 of the extruded stent 101. Any means for coating as known in the art may be utilized, including dip coating or spray coating. Preferably, the stent 101 is coated on a mandrel with the stent 101 in its expanded state or at least in its partially expanded state. Alternatively, other structural variations to the inner and outer photodegradable layers 103 and 102 are contemplated. For example, stent 101 may be inserted into an outer photodegradable sleeve. An inner photodegradable sleeve may also be slid within the luminal space 104 of stent 101. Biodegradable sutures as known in the art (e.g., 3-0 or 4-0 polydiaxanone absorbable monofilament sutures as commercially made and sold by Ethicon) may be utilized to affix the outer and the inner photodegradable sleeves. Alternatively, only an outer photodegradable sleeve may be used. Alternatively, the photodegradable layers 103 and 102 may be coextruded with the biodegradable layer 101.

In addition to the tubular main body shown in the Figures, the biodegradable stent 101 may comprise any other stent architecture as known in the art. In one example, the stent 101 may be braided on a mandrel from any one of the above mentioned biodegradable materials. In another example, the stent 101 may be coiled. In still another embodiment, the tubular body may be formed entirely from the photodegradable material described above. When the stent 100 is no longer required (e.g., the condition causing the stricture or other obstruction has been successfully resolved), degradation of the protective photodegradable layers 102 and 103 can begin. UV light contacts the photodegradable layers 102 and 103, which causes the photodegradable layers 102 and 103 to become activated and thereafter degrade. Because the entire stent 100 is composed form the photodegradable layers 102 and 103, the entire stent 101 is disintegrated such that an additional biodegradation process does not occur. Additionally, having the stent 100 formed entirely from a photodegradable layer may be advantageous to disintegrate the inner photodegradable layer that may have built-up encrustration thereon. Disintegration of the inner photodegradable layer exposes a clean surface with no encrustration, which can extend the life of the stent 100.

The biodegradable stent 101 may also be loaded with one or more bioactives in a therapeutically effective amount along at least a portion of the outer surface 111. As used herein, the term "bioactive" refers to any pharmaceutically active agent that produces an intended therapeutic effect on the body to treat or prevent conditions or diseases. The bioactive may be loaded along any portion of stent 101. Preferably, the bioactive is loaded along the outer surface 111 of biodegradable stent 101. In such an embodiment, the outer photodegradable layer 102 may serve as both the elution carrier and the protective chemically inert layer. A "therapeutically-effective amount" as used herein is the minimal amount of a bioactive which is necessary to impart therapeutic benefit to a human or veterinary patient. For example, a "therapeutically effective amount" to a human or veterinary patient is such an amount which induces, ameliorates or otherwise causes an improvement in the pathological symptoms, disease progression or physiological conditions associated with or resistance to succumbing to a disorder, for example restenosis. Accordingly, elution of the bioactive can occur when the protective outer photodegradable layer 102 is degraded as described above with reference to Figures 3a-3c.

In one embodiment, the bioactive is an antithrombogenic agent. Devices comprising an antithrombogenic agent are particularly preferred for implantation in areas of the body that contact blood. An antithrombogenic agent is any agent that inhibits or prevents thrombus formation within a body vessel. Types of antithrombotic agents include anticoagulants, antiplatelets, and fibrinolytics. Examples of antithrombotics include but are not limited to anticoagulants such as thrombin, Factor Xa, Factor VIIa and tissue factor inhibitors; antiplatelets such as glycoprotein IIb/IIIa, thromboxane A2, ADP-induced glycoprotein IIb/IIIa, and phosphodiesterase inhibitors; and fibrinolytics such as plasminogen activators, thrombin activatable fibrinolysis inhibitor (TAFI) inhibitors, and other enzymes which cleave fibrin. Further examples of antithrombotic agents include anticoagulants such as heparin, low molecular weight heparin, covalent heparin, synthetic heparin salts, coumadin, bivalirudin (hirulog), hirudin, argatroban, ximelagatran, dabigatran, dabigatran etexilate, D-phenalanyl-L-poly-L-arginyl, chloromethy ketone, dalteparin, enoxaparin, nadroparin, danaparoid, vapiprost, dextran, dipyridamole, omega-3 fatty acids, vitronectin receptor antagonists, DX-9065a, CI-1083, JTV-803, razaxaban, BAY 59-7939, and LY-51,7717; antiplatelets such as eftibatide, tirofiban, orbofiban, lotrafiban, abciximab, aspirin, ticlopidine, clopidogrel, cilostazol, dipyradimole; fibrinolytics such as alfimeprase, alteplase, anistreplase, reteplase, lanoteplase, monteplase, tenecteplase, urokinase, streptokinase, or phospholipid encapsulated microbubbles; and other bioactive agents such as endothelial progenitor cells or endothelial cells.

Another example of an antithrombotic agent is a nitric oxide source such as sodium nitroprussiate, nitroglycerin, S-nitroso and N-nitroso compounds. In one embodiment, a material capable of releasing nitric oxide from blood-contacting surfaces can be delivered by the device of the invention.

Other examples of bioactive agents suitable for inclusion in the devices of the present invention include antiproliferative/antimitotic agents including natural products such as vinca alkaloids (vinblastine, vincristine, and vinorelbine), paclitaxel, rapamycin analogs, epidipodophyllotoxins (etoposide, teniposide), antibiotics (dactinomycin (actinomycin D) daunorubicin, doxorubicin and idarubicin), anthracyclines, mitoxantrone, bleomycins, plicamycin (mithramycin) and mitomycin, enzymes (for example, L-asparaginase which systemically metabolizes L-asparagine and deprives cells which do not have the capacity to synthesize their own asparagine); antiplatelet agents such as (GP) II.sub.b/III.sub.a inhibitors and vitronectin receptor antagonists; antiproliferative/antimitotic alkylating agents such as nitrogen mustards (mechlorethamine, cyclophosphamide and analogs, melphalan, chlorambucil), ethylenimines and methylmelamines (hexamethylmelamine and thiotepa), alkyl sulfonates-busulfan, nirtosoureas (carmustine (BCNU) and analogs, streptozocin), trazenes-dacarbazinine (DTIC); antiproliferative/antimitotic antimetabolites such as folic acid analogs (methotrexate), pyrimidine analogs (fluorouracil, floxuridine, and cytarabine), purine analogs and related inhibitors (mercaptopurine, thioguanine, pentostatin and 2-chlorodeoxyadenosine{cladribine}); platinum coordination complexes (cisplatin, carboplatin), procarbazine, hydroxyurea, mitotane, aminoglutethimide; hormones (i.e. estrogen); anticoagulants (heparin, synthetic heparin salts and other inhibitors of thrombin); fibrinolytic agents (such as tissue plasminogen activator, streptokinase and urokinase), aspirin, dipyridamole, ticlopidine, clopidogrel, abciximab; antimigratory; antisecretory (breveldin); anti-inflammatory: such as adrenocortical steroids (cortisol, cortisone, fludrocortisone, prednisone, prednisolone, 6.alpha.-methylprednisolone, triamcinolone, betamethasone, and dexamethasone), non-steroidal agents (salicylic acid derivatives i.e. aspirin; para-aminophenol derivatives i. e. acetaminophen; indole and indene acetic acids (indomethacin, sulindac, and etodalac), heteroaryl acetic acids (tolmetin, diclofenac, and ketorolac), arylpropionic acids (ibuprofen and derivatives), anthranilic acids (mefenamic acid, and meclofenamic acid), enolic acids (piroxicam, tenoxicam, phenylbutazone, and oxyphenthatrazone), nabumetone, gold compounds (auranofin, aurothioglucose, gold sodium thiomalate); immunosuppressives (cyclosporine, tacrolimus (FK-506), sirolimus (rapamycin), tacrolimus, everolimus, azathioprine, mycophenolate mofetil); angiogenic agents: vascular endothelial growth factor (VEGF), fibroblast growth factor (FGF); angiotensin receptor blockers; nitric oxide and nitric oxide donors; antisense oligionucleotides and combinations thereof; cell cycle inhibitors, mTOR inhibitors, and growth factor receptor signal transduction kinase inhibitors; retenoids; cyclin/CDK inhibitors; endothelial progenitor cells (EPC); angiopeptin; pimecrolimus; angiopeptin; HMG co-enzyme reductase inhibitors (statins); metalloproteinase inhibitors (batimastat); protease inhibitors; antibodies, such as EPC cell marker targets, CD34, CD133, and AC 133/CD133; Liposomal Biphosphate Compounds (BPs), Chlodronate, Alendronate, Oxygen Free Radical scavengers such as Tempamine and PEA/NO preserver compounds, and an inhibitor of matrix metalloproteinases, MMPI, such as Batimastat. Still other bioactive agents that can be incorporated in or coated on a frame include a PPAR .alpha.-agonist, a PPAR .delta. agonist and RXR agonists, as disclosed in published U.S. Patent Application US2004/0073297 to Rohde etal.. In another embodiment, the bioactive is paclitaxel, rapamycin, a rapamycin derivative, an antisense oligonucleotide, or a mTOR.

The above mentioned hybrid stent 100 overcomes the problems associated with premature biodegradation of biodegradable stents by implementing a protective outer photodegradable layer over a biodegradable stent. By avoiding premature biodegradation, the stent 100 is able to exert sufficient radial strength at the target body lumen for the desired time period. Accordingly, the patency of the body lumen is achieved. When the stent 100 is no longer needed, the photodegradable layers 102 and 103 can be UV degraded so as to allow the stent body 101 to undergo biodegradation. The autonomous biodegradation advantageously eliminates an additional procedure typically required for removing the stent 101.

## Claims

1. A hybrid degradable stent comprising:
a generally tubular main body comprising an inner diameter and an outer diameter, wherein the tubular body is formed from a biodegradable material; and
a photodegradable layer disposed over at least a portion of the inner diameter and the outer diameter of the biodegradable tubular body, wherein the photodegradable layer is formed from a photodegradable material that is chemically inert to bodily fluids contained at an implanted site within the patient, the photodegradable layer selectively adapted to be activated from a chemically inert state to a photodegradable state, wherein the photodegradable state initiates degradation of the photodegradable layer so as to expose the portion of the biodegradable material over which the photodegradable layer is disposed so as to begin biodegradation of the biodegradable material.

2. The hybrid degradable stent of claim 1, wherein the photodegradable layer is selectively adapted to be activated from the chemically inert state to the photodegradable state by a ultraviolet (UV) light-irradiating system.

3. The hybrid degradable stent of claim 2, wherein the light-irradiating system comprises a ultraviolet (UV) light source and an optical fiber section, the optical fiber section comprising a proximal section in communication with the UV light source and a distal section in communication with at least one of the inner diameter and the outer diameter of the tubular body, wherein the optical fiber section is adapted to propagate and transmit UV light from the proximal section to the distal section, and thereafter irradiate light from the distal section to the tubular body, preferably wherein the optical fiber comprises a lens surface for redirecting the UV light.

4. The hybrid degradable stent according to any preceding claim, wherein the photodegradable material comprises a UV photodegradable ketocarbonyl containing polymer.

5. The hybrid degradable stent of claim 4, wherein the photodegradable material further comprises a synthetic polymer formed from a vinylidene monomer.

6. The hybrid degradable stent of claim 5, wherein the photodegradable ketocarbonyl comprises a chemical composition ranging from about 0.01 wt% to about 5 wt%, based upon the total weight of the synthetic polymer

7. The hybrid degradable stent according to any preceding claim, wherein the biodegradable tubular main body comprises a bioactive loaded therewithin.

8. The hybrid degradable stent according to any preceding claim, wherein the photodegradable layer is nonporous and extends along an entire length of the inner diameter and the outer diameter of the biodegradable tubular main body.

9. A degradable stent kit comprising:
a generally biodegradable tubular body comprising a proximal end and a distal end, and a lumen extending from the proximal end to the distal end, the body further comprising a photodegradable material disposed over at least a portion of the biodegradable tubular body, the photodegradable material being chemically inert when initially deployed into a body lumen of a patient; and
a light-irradiating system configured to activate the photodegradable material from the chemically inert state to a photodegradable state, the light-irradiating system comprising a light source and a fiber section, the fiber section comprising a proximal section in communication with the light source and a distal section in communication with the tubular body, wherein the fiber section is adapted to propagate ultraviolet (UV) light from the proximal section to the distal section and thereafter irradiate ultraviolet (UV) light from the distal section to the photodegradable material.

10. The kit of claim 9, wherein the fiber section is an optical fiber.

11. The kit of claim 9 or 10, wherein the light-irradiating system further comprises an intensity control to regulate the amount of UV light to be delivered to the optical fiber.

12. The kit to any of claims 9-10, wherein the tubular body is formed entirely from the photodegradable material.

13. The kit according to any of claims 9-12, wherein the light-irradiating system further comprises a wavelength tuning control for selecting UV light of a suitable wavelength to interact with the photodegradable material.

14. The kit according to any of claims 9-13, wherein the optical fiber comprises a means for selectively directing the UV light waves onto the photodegradable material.

15. The kit according to any of claims 9-14, wherein the photodegradable layer is disposed over the entire tubular body.

## Patentansprüche

1. Abbaubarer Hybrid-Stent, umfassend:
einen allgemein röhrenförmigen Hauptkörper, der einen Innendurchmesser und einen Außendurchmesser umfasst, worin der röhrenförmige Körper aus einem biologisch abbaubaren Material geformt ist; und
eine durch Licht abbaubare Schicht, die über zumindest einem Teil des Innendurchmessers und des Außendurchmessers des biologisch abbaubaren röhrenförmigen Körpers angeordnet ist, worin die durch Licht abbaubare Schicht aus einem durch Licht abbaubaren Material geformt ist, das gegenüber Körperflüssigkeiten, die an einer Implantationsstelle in dem Patienten enthalten sind, chemisch inert ist, wobei die durch Licht abbaubare Schicht selektiv ausgelegt ist, aus einem chemisch inerten Zustand in einen durch Licht abbaubaren Zustand aktiviert zu werden, worin der durch Licht abbaubare Zustand den Abbau der durch Licht abbaubaren Schicht einleitet, um den Abschnitt des biologisch abbaubaren Materials, über dem die durch Licht abbaubare Schicht angeordnet ist, freizulegen, damit der biologische Abbau des biologisch abbaubaren Materials beginnen kann.

2. Abbaubarer Hybrid-Stent nach Anspruch 1, worin die durch Licht abbaubare Schicht selektiv ausgelegt ist, durch ein ultraviolettes (UV) Lichtbestrahlungssystem aus dem chemisch inerten Zustand in den durch Licht abbaubaren Zustand aktiviert zu werden.

3. Abbaubarer Hybrid-Stent nach Anspruch 2, worin das Lichtbestrahlungssystem eine ultraviolette (UV) Lichtquelle und einen Lichtleiter-Abschnitt umfasst, wobei der Lichtleiterabschnitt einen proximalen Abschnitt, der mit der UV-Lichtquelle in Verbindung steht, und einen distalen Abschnitt umfasst, der zumindest mit dem Innendurchmesser und/oder mit dem Außendurchmesser des röhrenförmigen Körpers in Verbindung steht, worin der Lichtleiter-Abschnitt ausgelegt ist, UV-Licht zu verbreiten und von dem proximalen Abschnitt zu dem distalen Abschnitt zu übertragen und danach Licht von dem distalen Abschnitt auf den röhrenförmigen Körper abzustrahlen, worin vorzugsweise der Lichtleiter eine Linsenfläche zur Umleitung des UV-Lichts umfasst.

4. Abbaubarer Hybrid-Stent nach einem der vorhergehenden Ansprüche, worin das durch Licht abbaubare Material ein durch UV-Licht abbaubares Ketocarbonyl enthaltendes Polymer umfasst.

5. Abbaubarer Hybrid-Stent nach Anspruch 4, worin das durch Licht abbaubare Material ferner ein synthetisches Polymer umfasst, das aus einem Vinyliden-Monomer geformt ist.

6. Abbaubarer Hybrid-Stent nach Anspruch 5, worin das durch Licht abbaubare Ketocarbonyl eine chemische Zusammensetzung im Bereich von ungefähr 0,01 Gew.-% bis ungefähr 5 Gew.-%, auf Basis des Gesamtgewichts des synthetischen Polymers umfasst.

7. Abbaubarer Hybrid-Stent nach einem der vorhergehenden Ansprüche, worin der biologisch abbaubare röhrenförmige Hauptkörper ein darin geladenes biologisch wirksames Mittel umfasst.

8. Abbaubarer Hybrid-Stent nach einem der vorhergehenden Ansprüche, worin die durch Licht abbaubare Schicht nicht porös ist und sich entlang einer gesamten Länge des Innendurchmessers und des Außendurchmessers des biologisch abbaubaren schlauchförmigen Hauptkörpers erstreckt.

9. Abbaubares Stent-Kit, umfassend:
einen allgemein biologisch abbaubaren röhrenförmigen Körper, der ein proximales Ende und ein distales Ende und ein sich von dem proximalen Ende zu dem distalen Ende erstreckendes Lumen umfasst, wobei der Körper ferner ein durch Licht abbaubares Material umfasst, das über zumindest einem Teil des biologisch abbaubaren röhrenförmigen Körpers angeordnet ist, wobei das durch Licht abbaubare Material chemisch inert ist, wenn es anfänglich in einem Körperlumen eines Patienten eingesetzt wird; und
ein Lichtbestrahlungssystem, das zur Aktivierung des durch Licht abbaubaren Materials aus dem chemisch inerten Zustand in einen durch Licht abbaubaren Zustand ausgelegt ist, wobei das Lichtbestrahlungssystem eine Lichtquelle und einen Faserabschnitt umfasst, wobei der Faserabschnitt einen proximalen Abschnitt, der mit der Lichtquelle in Verbindung steht, und einen distalen Abschnitt, der mit dem röhrenförmigen Körper in Verbindung steht, aufweist, worin der Faserabschnitt ausgelegt ist, ultraviolettes (UV) Licht von dem proximalen Abschnitt zu dem distalen Abschnitt zu verbreiten und danach ultraviolettes (UV) Licht von dem distalen Abschnitt auf das durch Licht abbaubare Material abzustrahlen.

10. Kit nach Anspruch 9, worin der Faserabschnitt ein Lichtleiter ist.

11. Kit nach Anspruch 9 oder 10, worin das Lichtbestrahlungssystem ferner eine Intensitätskontrolle zur Regulierung der Menge des UV-Lichts, das an den Lichtleiter abgegeben werden soll, umfasst.

12. Kit nach einem der Ansprüche 9-10, worin der röhrenförmige Körper vollständig aus dem durch Licht abbaubaren Material geformt ist.

13. Kit nach einem der Ansprüche 9-12, worin das Lichtbestrahlungssystem ferner eine Wellenlängen-Abstimmungskontrolle zur Wahl von UV-Licht mit einer geeigneten Wellenlänge zur Interaktion mit dem durch Licht abbaubaren Material umfasst.

14. Kit nach einem der Ansprüche 9-13, worin der Lichtleiter ein Mittel zur selektiven Leitung der UV-Lichtwellen auf das durch Licht abbaubare Material umfasst.

15. Kit nach einem der Ansprüche 9-14, worin die durch Licht abbaubare Schicht über dem gesamten röhrenförmigen Körper angeordnet ist.

## Revendications

1. Endoprothèse vasculaire dégradable hybride, comprenant:
un corps principal essentiellement tubulaire présentant un diamètre intérieur et un diamètre extérieur, dans lequel le corps tubulaire est formé à partir d'un matériau biodégradable; et
une couche photodégradable qui est disposée sur au moins une partie du diamètre intérieur et du diamètre extérieur du corps tubulaire biodégradable, dans lequel la couche photodégradable est formée à partir d'un matériau photodégradable qui est chimiquement inerte aux fluides corporels contenus dans un site implanté à l'intérieur du patient, la couche photodégradable étant adaptée de façon sélective pour être activée d'un état chimiquement inerte à un état photodégradable, dans lequel l'état photodégradable amorce une dégradation de la couche photodégradable de manière à exposer la partie du matériau biodégradable sur laquelle la couche photodégradable est disposée de manière à commencer la biodégradation du matériau biodégradable.

2. Endoprothèse vasculaire dégradable hybride selon la revendication 1, dans laquelle la couche photodégradable est adaptée de façon sélective pour être activée de l'état chimiquement inerte à l'état photodégradable par un système de rayonnement de lumière ultraviolette (UV).

3. Endoprothèse vasculaire dégradable hybride selon la revendication 2, dans laquelle le système de rayonnement de lumière comprend une source de lumière ultraviolette (UV) et une section de fibre optique, la section de fibre optique comprenant une section proximale en communication avec la source de lumière UV, et une section distale en communication avec au moins un parmi le diamètre intérieur et le diamètre extérieur du corps tubulaire, dans laquelle la section de fibre optique est adaptée pour propager et transmettre une lumière UV à partir de la section proximale jusqu'à la section distale, et rayonner ensuite la lumière à partir de la section distale jusqu'au corps tubulaire, de préférence dans laquelle la fibre optique comprend une surface de lentille pour rediriger la lumière UV.

4. Endoprothèse vasculaire dégradable hybride selon l'une quelconque des revendications précédentes, dans laquelle le matériau photodégradable comprend un polymère contenant un cétocarbonyle photodégradable aux UV.

5. Endoprothèse vasculaire dégradable hybride selon la revendication 4, dans laquelle le matériau photodégradable comprend en outre un polymère synthétique qui est formé à partir d'un monomère de vinylidène.

6. Endoprothèse vasculaire dégradable hybride selon la revendication 5, dans laquelle le cétocarbonyle photodégradable présente une composition chimique comprise dans une gamme d'environ 0,01 % en poids à environ 5 % en poids, sur la base du poids total du polymère synthétique.

7. Endoprothèse vasculaire dégradable hybride selon l'une quelconque des revendications précédentes, dans laquelle le corps principal tubulaire biodégradable comprend un agent bioactif chargé à l'intérieur de celui-ci.

8. Endoprothèse vasculaire dégradable hybride selon l'une quelconque des revendications précédentes, dans laquelle la couche photodégradable est non poreuse et s'étend le long de la totalité de la longueur du diamètre intérieur et du diamètre extérieur du corps principal tubulaire biodégradable.

9. Kit d'endoprothèse vasculaire dégradable, comprenant:
un corps tubulaire essentiellement biodégradable présentant une extrémité proximale et une extrémité distale, et une lumière qui s'étend à partir de l'extrémité proximale jusqu'à l'extrémité distale, le corps comprenant en outre un matériau photodégradable qui est disposé sur au moins une partie du corps tubulaire biodégradable, le matériau photodégradable étant chimiquement inerte lorsqu'il est initialement déployé dans une lumière corporelle d'un patient; et
un système de rayonnement de lumière qui est configuré pour activer le matériau photodégradable de l'état chimiquement inerte à un état photodégradable, le système de rayonnement de lumière comprenant une source de lumière et une section de fibre, la section de fibre comprenant une section proximale qui est en communication avec la source de lumière et une section distale qui est en communication avec le corps tubulaire, dans lequel la section de fibre est adaptée pour propager une lumière ultraviolette (UV) à partir de la section proximale jusqu'à la section distale, et rayonner ensuite la lumière ultraviolette (UV) à partir de la section distale jusqu'au matériau photodégradable.

10. Kit selon la revendication 9, dans lequel la section de fibre est une fibre optique.

11. Kit selon la revendication 9 ou 10, dans lequel le système de rayonnement de lumière comprend en outre une commande d'intensité pour réguler la quantité de lumière UV à délivrer à la fibre optique.

12. Kit selon l'une quelconque des revendications 9 ou 10, dans lequel le corps tubulaire est formé entièrement à partir du matériau photodégradable.

13. Kit selon l'une quelconque des revendications 9 à 12, dans lequel le système de rayonnement de lumière comprend en outre une commande de réglage de longueur d'onde pour sélectionner une lumière UV d'une longueur d'onde appropriée pour interagir avec le matériau photodégradable.

14. Kit selon l'une quelconque des revendications 9 à 13, dans lequel la fibre optique comprend des moyens pour diriger de façon sélective les ondes de lumière UV sur le matériau photodégradable.

15. Kit selon l'une quelconque des revendications 9 à 14, dans lequel la couche photodégradable est disposée sur la totalité du corps tubulaire.
